# EUROPEAN PATENT APPLICATION

(11) **EP 4 756 031 A1**
(43) Date of publication of application: **10.06.2026**
(21) Application number: 24897983.3
(22) Date of filing: 18.11.2024
(51) Int. Cl.: C12N 15/67, C12N 15/77, C12N 15/70, C12N 15/75

(54) **NOVEL PROMOTER AND USE THEREOF**

(30) Priority: 27.11.2023 KR 20230166694
(71) Applicant: CJ Cheiljedang Corporation, Seoul 04560 (KR)
(72) Inventor: YOON, Jeong-Hye, Seoul 04560 (KR); PARK, Goun, Seoul 04560 (KR); BAE, Jee Yeon, Seoul 04560 (KR); KIM, Hyung Joon, Seoul 04560 (KR)
(74) Representative: Meissner Bolte Partnerschaft mbB
(86) International application number: PCT/KR2024/018165
(87) International publication number: WO 2025/116387

(57) **Abstract**

Provided are a novel promoter and a method of producing a target product using the same. The polynucleotide of the present disclosure has promoter activity, and may efficiently control the expression and activity of a target gene in a microorganism, and may be usefully applied in efficiently producing the target product.

## Description

### [Technical Field]

The present disclosure relates to a novel promoter and a method of producing a target product using the same.

### [Background Art]

In order to produce target substances such as amino acids or useful substances with a wide range of applications such as feeds, medicines, foods, etc., with high potency using microorganisms, efforts have been continuously made to manipulate genes in biosynthetic pathways and/or to introduce foreign genes (US 9109242 B2). One of these methods is a method of inducing overexpression of a target gene in microorganisms, which requires a highly efficient gene expression system. Promoters are one of the most critical elements involved in the gene expression system, and therefore, it is necessary to develop useful promoters.

The tac promoter derived from E. *coli* is widely known as a strong promoter, and in the case of *coryneform* bacteria, strong promoters have been developed by modifying promoters of their own genes (Gene, 102, 93-98, 1991; Microbiology, 142, 1297-1309, 1996). Meanwhile, the general structure of a promoter sequence for expressing a gene in *coryneform* bacteria is not known, unlike the structures of other industrial microorganisms, such as E. *coli* or *Bacillus subtilis,* etc. For this reason, promoters have been developed as follows: a promoter region of a gene that is resistant to an antibiotic, such as chloramphenicol, is removed, and a chromosomal DNA separated from *coryneform* bacteria, which is cleaved using a suitable restriction enzyme, is introduced thereto, and the resultant is used to transform *coryneform* bacteria to produce a transformed strain, which antibiotic resistance is measured. In addition, various promoter searches have been conducted to overexpress foreign genes in microorganisms of the genus *Bacillus,* and these promoters are used in the production of enzymes for foods, pharmaceutical and other industrial purposes. Many vector systems using promoters for expression of alpha-amylase, protease, and lipase genes in various microorganisms of the genus *Bacillus* are still being developed to this day (Schumann 2007. Adv. Appl. Microbiol. 153:813-821).

However, there is still a need for a system exhibiting high expression efficiency in various microorganisms, i.e., microorganisms of the genus *Escherichia,* microorganisms of the genus *Corynebacterium,* or microorganisms of the genus *Bacillus,* and accordingly, it is still necessary to develop a promoter for general use.

### [Disclosure]

### [Technical Problem]

The present inventors found that the introduction of a novel promoter into a microorganism may increase the expression and activity of a gene operably linked thereto, and the novel promoter may be used as a promoter for general use, rather than as a promoter limited to a specific gene, to be usefully applied in efficiently producing a target product that is influenced by the promoter and gene, thereby completing the present disclosure.

### [Technical Solution]

An object of the present disclosure is to provide a polynucleotide having promoter activity, the polynucleotide comprising any one polynucleotide sequence selected from the group consisting of SEQ ID NOS: 1 to 3.

Another object of the present disclosure is to provide an expression cassette comprising the polynucleotide and a target gene.

Still another object of the present disclosure is to provide a microorganism comprising the polynucleotide or the expression cassette.

Still another object of the present disclosure is to provide a method of producing a target product, the method comprising the step of culturing the microorganism in a medium.

### [Advantageous Effects]

A novel promoter of the present disclosure is introduced into a microorganism to increase the expression and activity of a gene operably linked thereto, and the novel promoter may be used as a promoter for general use, rather than as a promoter limited to a specific gene, thereby being usefully applied in efficiently producing a target product that is influenced by the promoter and gene.

### [Brief Description of the Drawing]

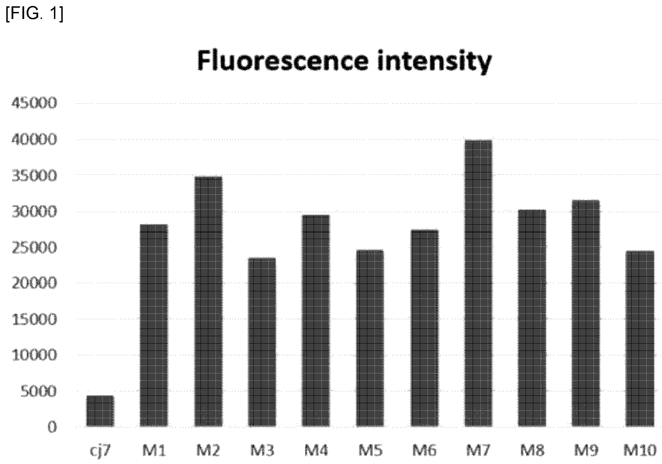

FIG. 1 is a graph showing the green fluorescence intensity in a mutant strain comprising a cj7 promoter variant operably linked to a green fluorescent protein gene according to one embodiment.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

One aspect of the present disclosure provides a polynucleotide having promoter activity, the polynucleotide comprising any one polynucleotide sequence selected from the group consisting of SEQ ID NOS: 1 to 3.

As used herein, the term "polynucleotide" refers to a DNA strand having a certain length or more as a polymer of nucleotides in which nucleotide monomers are connected in a long chain by covalent bonds.

As used herein, the term "promoter" refers to an untranslated nucleotide sequence upstream of a coding region, which comprises a binding site for polymerase and has the activity of initiating the transcription of a target gene into mRNA, i.e., a DNA region to which polymerase binds to initiate the transcription of a gene. The promoter may be located at the 5' region of an mRNA transcription start site.

As used herein, the term "polynucleotide having promoter activity" refers to a DNA region which comprises a binding site for RNA polymerase or enhancer, etc. for expression of a gene operably linked downstream of the promoter, i.e., a target gene, and exists near a transcription site of the target gene. With respect to the objects of the present disclosure, the polynucleotide may be used as a promoter for general use, and as compared with the existing promoter or endogenous promoter, in cells, the polynucleotide may regulate (e.g., increase or decrease) expression of a target gene operably linked thereto and production and/or activity of a protein encoded by the target gene, and it may regulate (e.g., increase or decrease) production and/or activity of a target product (a biologically active substance, e.g., one or more selected from the group consisting of amino acids, nucleic acids, vitamins, proteins, fatty acids, organic acids, etc.) where the target gene is involved in the production thereof, but is not limited thereto. The amino acids, nucleic acids, vitamins, proteins, fatty acids, or organic acids may also comprise metabolites thereof, precursors thereof, or derivatives thereof, but are not limited thereto.

The polynucleotide of the present disclosure may comprise any polynucleotide sequence without limitation, as long as it has promoter activity.

In the present disclosure, the polynucleotide having promoter activity, the polynucleotide comprising the polynucleotide sequence of SEQ ID NO: 1 to SEQ ID NO: 3, may be interchangeably with "polynucleotide" or "cj7.7 promoter (SEQ ID NO: 1)", "cj7.8 promoter (SEQ ID NO: 2)", or "cj7.9 promoter (SEQ ID NO: 3)", respectively, and in the present disclosure, the terms described above may be used interchangeably.

The polynucleotide may have any one polynucleotide sequence selected from SEQ ID NOS: 1 to 3, and may comprise any one polynucleotide sequence selected from SEQ ID NOS: 1 to 3, may essentially consist of any one polynucleotide sequence selected from SEQ ID NOS: 1 to 3, or may consist of any one polynucleotide sequence selected from SEQ ID NOS: 1 to 3.

Although described as 'a polynucleotide having a nucleotide sequence represented by a specific SEQ ID NO' or 'a polynucleotide comprising a nucleotide sequence represented by a specific SEQ ID NO' in the present disclosure, it is obvious that any polynucleotide having a nucleotide sequence with deletion, modification, substitution, or addition in part of the sequence may also be used in the present disclosure, as long as the polynucleotide may have activity identical or corresponding to that of the polynucleotide consisting of the nucleotide sequence of the corresponding SEQ ID NO. For example, as long as a polynucleotide has activity identical or corresponding to that of the polynucleotide, it does not exclude addition of a meaningless sequence upstream or downstream of the nucleotide sequence of the corresponding SEQ ID NO, a naturally occurring mutation, or a silent mutation thereof, and it is obvious that such a sequence addition or mutation also falls within the scope of the present disclosure.

The polynucleotide provided in the present disclosure may comprise the polynucleotide sequence of SEQ ID NO: 1, 2, or 3, and may refer to (a) a polynucleotide comprising or essentially comprising the polynucleotide sequence of SEQ ID NO: 1, 2, or 3, or a polynucleotide sequence complementary to the sequence, and/or (b) a polynucleotide comprising the polynucleotide sequence of SEQ ID NO: 1, 2, or 3, or a polynucleotide sequence having 60% or more, 70% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more homology or identity to a polynucleotide sequence complementary to the sequence while retaining the original function and/or the desired function of the polynucleotide.

For example, the original function and/or the desired function of the polynucleotide may be the function as the promoter. The polynucleotide has 'the function as the promoter and comprises the polynucleotide sequence of SEQ ID NO: 1, 2, or 3' may mean that when the polynucleotide is operably linked to a target gene to be used as a promoter, it does not exclude the case where mutations such as addition, and/or deletion, and/or substitution of nucleotides that may occur during the process of linking to the target gene, such as use of a restriction enzyme, may be induced (introduced) to the polynucleotide sequence of SEQ ID NO: 1, 2, or 3. In addition, the polynucleotide comprising the polynucleotide sequence of SEQ ID NO: 1, 2, or 3 and having the promoter function may comprise, without limitation, any polynucleotide that hybridizes under stringent conditions with all or a part of the polynucleotide sequence of SEQ ID NO: 1, 2, or 3 or a complementary sequence thereto and has promoter activity. SEQ ID NOS: 1 to 3 with promoter activity may be a mutant promoter of Pcj7 promoter having enhanced promoter activity, as compared to a known promoter, Pcj7 promoter of SEQ ID NO: 4 (US 7662943 B2).

Further, the polynucleotides provided in the present disclosure may be natural or non-natural, and may be non-natural, for example, chemically or recombinantly synthesized.

As used herein, homology and identity refer to a degree of relevance between two given nucleotide sequences, and may be expressed as a percentage.

The terms 'homology and identity' may often be used interchangeably with each other.

The sequence homology or identity of conserved polynucleotides may be determined by standard alignment algorithms and may be used with a default gap penalty established by the program being used. Substantially, homologous or identical sequences may generally hybridize under moderately or highly stringent conditions to the entire length of the sequence or at least about 50%, 60%, 70%, 80%, or 90% of the full-length. In hybridization of polynucleotides, polynucleotides comprising degenerate codon(s) instead of normal codon(s) are also considered.

Whether any two polynucleotide sequences have homology, similarity, or identity may be, for example, determined using a known computer algorithm such as the "FASTA" program using default parameters, as in Pearson et al (1988)[Proc. Natl. Acad. Sci. USA 85]: 2444. Alternatively, it may be determined by the Needleman Wunsch algorithm (Needleman and Wunsch, 1970, J. Mol. Biol. 48: 443-453), as performed using the Needleman program of the EMBOSS package (EMBOSS: The European Molecular Biology Open Software Suite, Rice et al., 2000, Trends Genet. 16: 276-277) (version 5.0.0 or later) (GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073)). For example, the homology, similarity, or identity may be determined using BLAST or ClustalW of the National Center for Biotechnology Information.

The homology, similarity, or identity of polynucleotides may be, for example, determined by comparing sequence information using, for example, the GAP computer program, such as Needleman et al. (1970), J Mol Biol.48: 443, as disclosed in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. Default parameters for the GAP program may comprise (1) a unary comparison matrix (comprising a value of 1 for identities and 0 for non-identities) and the weighted comparison matrix of Gribskov et al(1986) Nucl. Acids Res. 14: 6745, as disclosed in Schwartz and Dayhoff, eds., Atlas Of Protein Sequence And Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps. Therefore, the term "homology" or "identity", as used herein, represents relevance between sequences.

Further, the polynucleotide may comprise a probe that may be prepared from a known gene sequence, e.g., any polynucleotide sequence without limitation as long as it is capable of hybridizing with a sequence complementary to all or part of the above-described polynucleotide sequence under stringent conditions and retaining the identical activity. The "stringent conditions" refers to conditions under which specific hybridization between polynucleotides is allowed. Such conditions are specifically described in literature (e.g., J. Sambrook et al., supra). For example, the stringent conditions may comprise conditions under which genes having high homology or identity, genes having 40% or more, specifically 70% or more, 80% or more, 85% or more, 90% or more, more specifically 95% or more, much more specifically 97% or more, most specifically 99% or more homology or identity are hybridized with each other, and genes having homology or identity lower than the above are not hybridized with each other, or washing conditions of the common Southern hybridization, i.e., washing once, specifically, twice or three times at a salt concentration and a temperature corresponding to 60°C, 1XSSC, 0.1% SDS, specifically 60°C, 0.1XSSC, 0.1% SDS, and more specifically 68°C, 0.1XSSC, 0.1% SDS.

Hybridization requires that two polynucleotides have complementary sequences, although mismatches between bases are possible depending on the stringency of the hybridization. The term "complementary" is used to describe the relationship between nucleotide bases that may hybridize with each other. For example, with respect to DNA, adenine is complementary to thymine, and cytosine is complementary to guanine. Therefore, the present disclosure may also comprise isolated polynucleotide fragments complementary to the entire sequence as well as polynucleotide sequences substantially similar thereto.

Specifically, polynucleotides having homology or identity may be detected using the hybridization conditions comprising a hybridization step at a Tm value of 55°C under the above-described conditions. Further, the Tm value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and variables are well known in the art (see Sambrook et al., supra, 9.50-9.51, 11.7-11.8).

The polynucleotide having the promoter activity of the present disclosure may be used as a promoter.

The promoter may be located at the 5' region of an mRNA transcription start site.

The promoter may have increased or decreased promoter activity, as compared with existing promoters. In other words, the promoter may increase or decrease expression and/or activity of a protein encoded by a target gene as well as expression of the target gene in host cells. With respect to the objects of the present disclosure, the target gene of which expression is enhanced or weakened may be changed according to a product to be produced, and the promoter may be used as a general-use promoter for enhancing or weakening the target gene.

The "target gene" refers to a gene whose expression is regulated by the promoter sequence of the present disclosure with respect to the objects of the present disclosure. A protein encoded by the target gene may be expressed as a "target protein", and a gene encoding the "target protein" may be expressed as a "target gene".

The target gene may be used without limitation as long as it is a gene whose expression is regulated by the promoter sequence of the present disclosure, and may be a foreign gene.

The "foreign gene" refers to a gene that is non-natively (non-naturally) comprised in microorganisms. For example, the foreign gene may be (a) a (foreign or artificial) gene that may not be naturally found in a microorganism, (b) a gene whose transcription or translation in a microorganism results in an unnatural amount (more or less than the amount naturally present) even though the (endogenous) gene may be naturally found in the microorganism, (c) a gene whose sequence is different even though the gene encodes a protein sequence endogenously present in a microorganism, and/or (d) a combination of two or more in a microorganism.

Further, the polynucleotide encoding the target protein may undergo various modifications in the coding region within the scope that does not change the polypeptide sequence, due to codon degeneracy or in consideration of the codons preferred in an organism in which the polynucleotide is to be expressed. The polynucleotide sequence is as described above.

Another aspect of the present disclosure provides the polynucleotide; an expression cassette comprising the polynucleotide and a target gene and/or a vector comprising the polynucleotide or the expression cassette.

As used herein, the term "expression cassette" refers to a unit cassette which comprises a promoter and a target gene, and is capable of expressing the target gene operably linked to the promoter. Various factors that are able to aid the efficient expression of the target gene may be comprised inside or outside of the gene expression cassette. Generally, the gene expression cassette may comprise a transcription termination signal, a ribosome-binding domain, and a translation termination signal, in addition to the promoter operably linked to the target gene.

As used herein, the term "operably linked" means that the polynucleotide having promoter activity of the present disclosure is functionally linked to the gene sequence to initiate and mediate transcription of the target gene. Operable linking may be prepared using a genetic recombination technique known in the art, and site-specific DNA cleavage and ligation may be performed by using a restriction enzyme and ligase known in the art, but is not limited thereto.

As used herein, the term "vector", which is an artificial DNA molecule having a genetic material capable of expressing a target gene in a suitable host, refers to a DNA construct comprising the polynucleotide having promoter activity or an additional suitable gene expression regulatory sequence. The vector may be a vector which may integrate the polynucleotide having the promoter activity into a host cell, but is not limited thereto. Alternatively, the vector may be a vector comprising a target gene. The regulatory sequence may comprise an additional promoter capable of initiating transcription, any operator sequence for regulating such transcription, a sequence encoding a suitable mRNA ribosome-binding site, and/or a sequence for regulating termination of transcription and translation. Once transformed into a suitable host cell, the vector may replicate or function independently from the host genome, or may integrate into the genome thereof.

The vector used in the present disclosure is not particularly limited, and any vector known in the art may be used. Examples of vectors commonly used comprise natural or recombinant plasmids, cosmids, viruses, and bacteriophages. For example, as phage vectors or cosmid vectors, pWE15, M13, MBL3, MBL4, IXII, ASHII, APII, t10, t11, Charon4A, Charon21A, etc. may be used. As plasmid vectors, those based on pBR, pUC, pBluescriptll, pGEM, pTZ, pCL and pET, etc. may be used. Specifically, pDZ, pACYC177, pACYC184, pCL, pECCG117, pUC19, pBR322, pMW118, pCC1BAC vector, etc. may be used.

For example, the polynucleotide having the promoter activity and/or target gene (polynucleotide) may be inserted into a chromosome through a vector for intracellular chromosome insertion. Insertion of the polynucleotide and/or target gene (polynucleotide) into the chromosome may be performed by any method known in the art, for example, homologous recombination, but is not limited thereto. A selection marker for the confirmation of chromosome insertion may be further comprised. The selection marker is for selecting the cells transformed with vectors, i.e., for confirming the insertion of the polynucleotide and/or target gene (polynucleotide) molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

For example, in the recombinant vector, when the gene encoding the target protein is a fusion gene comprising genes each encoding two or more proteins, all of the genes comprised in the fusion gene may be designed to be under the control of one polynucleotide (promoter), or one or more may be designed to be under the control of a separate polynucleotide. For example, the recombinant vector may comprise the polynucleotide having promoter activity and one gene or two or more (e.g., 2, 3, 4, 5, 6, 7, 8, 9, or 10) genes operably linked to the polynucleotide, and when the vector comprises two or more genes, it may comprise one polynucleotide having the promoter activity (i.e., the two or more genes are under the control of one promoter) or two or more (in this case, the polynucleotide may be comprised in a number equal to or less than the number of genes so that one or more of the two or more genes are under the control of a separate promoter), but is not limited thereto.

As used herein, the term "transformation" means that a vector comprising a target polynucleotide (gene) is introduced into a host cell so that the polynucleotide may be expressed in the host cell. The transformed vector may be located regardless of the position, either by being inserted into the chromosome of the host cell or located outside the chromosome as long as the polynucleotide may be expressed in the host cell. Further, the polynucleotide comprises DNA or RNA. The polynucleotide may be introduced in any form as long as it may be introduced into a host cell and expressed. For example, the polynucleotide may be introduced into a host cell in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Still another aspect of the present disclosure provides a microorganism comprising the polynucleotide having the promoter activity of the present disclosure, or the expression cassette comprising the polynucleotide and a target gene.

The polynucleotide having the promoter activity and the expression cassette are as described above.

The expression cassette and the vector comprising the same may be introduced into a microorganism by transformation.

As used herein, the term "microorganism" comprises all of wild-type microorganisms or naturally or artificially genetically modified microorganisms, and is a concept comprising microorganisms in which a specific mechanism is weakened or strengthened due to an insertion of a foreign gene or an activity enhancement or weakening of an endogenous gene. In the present disclosure, the microorganism may comprise any microorganism without limitation as long as it is a microorganism into which the polynucleotide having the promoter activity of the present disclosure is introduced.

The microorganism comprising the polynucleotide of the present disclosure may be a microorganism expressing a target gene using one of the nucleotide sequences of SEQ ID NO: 1 to SEQ ID NO: 3 as a promoter, or having the ability to produce a target product in which the target gene is involved in its production, but is not limited thereto. The microorganism may be a microorganism naturally expressing the target gene or having the target product-producing ability or a microorganism prepared by providing the target gene-expressing ability or the target product-producing ability for a parent strain not expressing the target gene or not having the target product-producing ability, but is not limited thereto.

The microorganism may be, for example, a cell or microorganism that is transformed with the vector comprising the polynucleotide having the promoter activity of the present disclosure and the target gene to express the target gene. With respect to the objects of the present disclosure, the host cell or microorganism may be any microorganism as long as it is able to produce the target product by comprising the target gene.

As used herein, the term "microorganism producing the target protein or target product" comprises all wild-type microorganisms or naturally or artificially genetically modified microorganisms, and it may be a microorganism in which a specific mechanism is weakened or strengthened due to an insertion of a foreign gene or an activity enhancement or inactivation of an endogenous gene, and it may be a microorganism comprising a genetic modification for the production of a target protein or product. With respect to the objects of the present disclosure, the microorganism producing the target protein or target product may be a microorganism characterized in that the ability to produce the target protein or target product is increased by comprising the polynucleotide having the promoter activity of the present disclosure. Specifically, in the present disclosure, the microorganism producing the target protein or target product or the microorganism having the ability to produce the target protein or target product may be a microorganism in which a part of genes in the target protein or target product biosynthetic pathway is strengthened or weakened or a part of genes in the target protein or target product degradation pathway is strengthened or weakened.

Specifically, the microorganism of the present disclosure may be a microorganism of the genus *Escherichia,* the genus *Erwinia,* the genus *Serratia,* the genus *Providencia,* the genus *Corynebacterium,* the genus *Bacillus,* or the genus *Brevibacterium,* specifically, a microorganism of the genus *Corynebacterium,* the genus *Escherichia,* or the genus *Bacillus,* for example, *Corynebacterium glutamicum, Bacillus subtilis,* or *Escherichia coli,* but may comprise any microorganism without limitation, as long as it is a microorganism in which the polynucleotide having the promoter activity of the present disclosure may act as a promoter.

As used herein, the term "target product" refers to a biologically active substance to be produced or whose production is to be controlled (increased or decreased) using the polynucleotide provided in the present disclosure, the expression cassette comprising the polynucleotide and the target gene, the vector comprising the polynucleotide or the expression cassette, and/or the microorganism, and is a concept comprising not only the biologically active substance to be ultimately produced but also the target protein that may be produced by the microorganism. For example, it may be the target protein itself encoded by the target gene, and/or all biologically active substances in which the target protein is involved in their production. The biologically active substances refer to any substance that is produced or derived from an organism (e.g., a cell) or that has a predetermined function within a living body or cell, for example, may be amino acids (glycine, alanine, valine, leucine, isoleucine, threonine, serine, cysteine, glutamine, methionine, aspartic acid, asparagine, glutamic acid, lysine, arginine, histidine, phenylalanine, tyrosine, tryptophan, proline, O-acetyl homoserine, etc.), nucleic acids, vitamins (vitamin A, B (B1, B2, B3, B5, B6, B7, B9, B12, etc.), C, D, E, K, etc.), proteins (the target protein and other proteins, e.g., hormones, growth factors, cytokines, immunoglobulins (antibodies), antigen proteins, receptors, ligands, functional fragments thereof (fragments with the desired function), fusion proteins in which two or more are fused, etc.), sugars (e.g., monosaccharides, disaccharides, polysaccharides, sugar alcohols, etc.), fatty acids (myristoleic acid, palmitoleic acid, sapienic acid, oleic acid, elaidic acid, vaccenic acid, linoleic acid, linolenic acid, linolelaidic acid arachidonic acid, eicosapentaenoic acid (EPA), erucic acid, docosahexaenoic acid (DHA), etc.), organic acids (lactic acid, citric acid, oxalic acid, uric acid, butyric acid, stearic acid, propionic acid, etc.), but are not limited thereto. In addition, when the biologically active substance is a substance in which the target protein is involved in its production, it may comprise, in addition to the above substances, metabolites thereof (polyhydroxyalkanoate (PHA), etc.), precursors thereof, and derivatives thereof maintaining biological activity thereof, etc.

As used herein, the term "target protein" refers to a protein involved in the production of the biologically active substance which is produced or whose production is regulated (e.g., increased or decreased) using the polynucleotide provided in the present disclosure, the expression cassette comprising the polynucleotide and the target gene, the vector comprising the polynucleotide or the expression cassette, and/or the microorganism. For example, the target protein may refer to the target protein itself encoded by the target gene.

For example, when the target protein is involved in the production of the target product, (1) the target protein may be one or more selected from the group consisting of proteins involved in at least one process or step of an intracellular production pathway (e.g., biosynthesis, metabolism, bioconversion, etc.), intracellular transport, and/or extracellular excretion pathway of target products (e.g., amino acids, nucleic acids, vitamins, other proteins than the target protein, etc.), for example, enzymes (various synthetases, lyases, kinases, carboxylases (e.g., pyruvate carboxylase, etc.), reductases, oxidases, decarboxylases, dehydrogenases, dehydratases, transferases, epimerases, etc.), intermediates, transport proteins, membrane proteins (channels, etc.), etc., but are not limited thereto, and (2) a target or protein whose expression (production) is to be increased, or a protein involved in the production of a biologically active substance which is produced or whose production is increased. The target gene may be a gene encoding the target protein described in (1) or (2). For example, the target gene may be, but is not limited to, a gene encoding a protein involved in the production of a predetermined biological substance, such as an amino acid, nucleic acid, or vitamin.

For example, a gene involved in the production of lysine among the amino acids may be one or more genes selected from genes encoding proteins (e.g., enzymes) involved in lysine biosynthesis. For example, the proteins involved in lysine biosynthesis may be one or more selected from the group consisting of dihydrodipicolinate synthase (dapA), aspartokinase III (lysC), dihydrodipicolinate reductase (dapB), diaminopimelate decarboxylase (lysA), diaminopimelate dehydrogenase (ddh), phosphoenolpyruvate carboxylase (ppc), aspartate semialdehyde dehydrogenase (asd), aspartate transaminase (aspC), diaminopimelate epimerase (dapF), tetrahydrodipicolinate succinylase (dapD), succinyl-diaminopimelate deacylase (dapE), and aspartase (aspA), etc., but are not limited thereto.

A gene involved in the production of threonine among the amino acids may be one or more genes selected from genes encoding proteins (e.g., enzymes) involved in threonine biosynthesis. For example, the proteins involved in threonine biosynthesis may be one or more selected from the group consisting of aspartokinase III (lysC), aspartate semialdehyde dehydrogenase (asd), aspartokinase I (thrA), homoserine kinase (thrB), threonine synthase (thrC), and aspartate aminotransferase (AspAT/ASAT/AAT), etc., but are not limited thereto.

A gene involved in the production of arginine among the amino acids may be one or more genes selected from genes encoding proteins (e.g., enzymes) involved in arginine biosynthesis. For example, the proteins involved in arginine biosynthesis may be one or more selected from the group consisting of N-acetylglutamate synthetase (argA), N-acetylglutamate kinase (argB), N-acetylglutamyl phosphate reductase (argC), acetylornithine transaminase (argD), acetylornithine deacetylase (argE), ornithine carbamoyl transferase (argF, argI), argininosuccinic acid synthetase (argG), argininosuccinic acid lyase (argH), ornithine acetyl transferase (argJ), and carbamoyl phosphate synthetase (carAB), etc., but are not limited thereto.

A gene involved in the production of valine among the amino acids may be one or more genes selected from genes encoding proteins (e.g., enzymes) involved in valine biosynthesis. For example, the proteins involved in valine biosynthesis may be one or more selected from the group consisting of acetohydroxy acid isomeroreductase (IIvC), dihydroxy-acid dehydratase (IIvD), and branched-chain amino acid aminotransferase (IIvE), etc., but are not limited thereto.

A gene involved in the production of histidine among the amino acids may be one or more genes selected from genes encoding proteins (e.g., enzymes) involved in histidine biosynthesis. For example, the proteins involved in histidine biosynthesis may be one or more selected from the group consisting of ATP phosphoribosyltransferase (hisG), phosphoribosyl-AMP cyclohydrolase (hisI), phosphoribosyl-ATP pyrophosphohydrolase (hisl), phosphoribosylformimino-5-aminoimidazole carboxamide ribotide isomerase (hisA), amidotransferase (hisH), histidinol phosphate aminotransferase (hisC), histidinol phosphatase (hisB), and histidinol dehydrogenase (hisD), etc., but are not limited thereto.

A gene involved in the production of cysteine among the amino acids may be one or more genes selected from genes encoding proteins (e.g., enzymes) involved in cysteine biosynthesis. For example, the proteins involved in cysteine biosynthesis may be one or more selected from the group consisting of serine acetyltransferase (cysE) and d-3-phosphoglycerate dehydrogenase (serA), etc., but are not limited thereto.

A gene involved in the production of serine among the amino acids may be one or more genes selected from genes encoding proteins (e.g., enzymes) involved in serine biosynthesis. For example, the proteins involved in serine biosynthesis may be one or more selected from the group consisting of 3-phosphoglycerate dehydrogenase (serA), phosphoserine transaminase (serC), and phosphoserine phosphatase (serB), etc., but are not limited thereto.

A gene involved in the production of glutamic acid among the amino acids may be one or more genes selected from genes encoding proteins (e.g., enzymes) involved in glutamic acid biosynthesis. For example, the proteins involved in glutamic acid biosynthesis may be one or more selected from the group consisting of glutamate dehydrogenase (gdhA), glutamine synthetase (glnA), glutamate synthetase (gltBD), isocitrate dehydrogenase (icdA), aconitate hydratase (acnA, acnB), citrate synthase (gltA), methyl citrate synthase gene (prpC), phosphoenolpyruvate carboxylase (ppc), pyruvate carboxylase (pyc), pyruvate dehydrogenase (aceEF, IpdA), pyruvate kinase (pykA, pykF), phosphoenolpyruvate synthase (ppsA), enolase (eno), phosphoglyceromutase (pgmA, pgml), phosphoglycerate kinase (pgk), glyceraldehyde-3-phophate dehydrogenase (gapA), triose phosphate isomerase (tpiA), fructose bisphosphate aldolase (fbp), phosphofructokinase (pfkA, pfkB), glucose phosphate isomerase (pgi), 6-phosphogluconic acid dehydratase (edd), 2-keto-3-deoxy-6-phosphogluconic acid aldolase (eda), and transhydrogenase, etc., but are not limited thereto.

A gene involved in the production of glutamine among the amino acids may be one or more genes selected from genes encoding proteins (e.g., enzymes) involved in glutamine biosynthesis. For example, the proteins involved in glutamine biosynthesis may be one or more selected from the group consisting of glutamate dehydrogenase (gdhA), glutamine synthetase (glnA), etc., but are not limited thereto.

A gene involved in the production of proline among the amino acids may be one or more genes selected from genes encoding proteins (e.g., enzymes) involved in proline biosynthesis. For example, the proteins involved in proline biosynthesis may be one or more selected from the group consisting of glutamate-5-kinase (proB), γ-glutamyl-phosphate reductase, pyrroline-5-carboxylate reductase (putA), etc., but are not limited thereto.

A gene involved in the production of nucleic acids may be one or more genes selected from genes encoding proteins (e.g., enzymes) involved in nucleic acid biosynthesis. For example, the proteins involved in nucleic acid biosynthesis may be one or more selected from the group consisting of amidophosphoribosyltransferase (purF), PRA-glycine ligase (purD), phosphoribosylaminoimidazolesuccinocarboxamide synthase (purC), bifunctional AICAR formyltransferase/IMP cyclohydrolase (purH), adenylosuccinate synthase (purA), adenylosuccinate lyase (purB), phosphoribosylaminoimidazole mutase (purE), and phosphoribosylaminoimidazole carboxylase (purK), etc., but are not limited thereto.

Still another aspect of the present disclosure provides a method of producing the target product, the method comprising the step of culturing the microorganism in a medium. Further, the method may further comprise the step of recovering the target product from the microorganism or the medium in which the microorganism is cultured.

The microorganism and the target product are as described above.

As used herein, the term "culturing" refers to growing the microorganism in appropriately and artificially controlled environmental conditions. In the present disclosure, the method of producing the target product using the microorganism comprising the polynucleotide may be performed using a method widely known in the art.

In the above method, the step of culturing the microorganism may be, but is not particularly limited to, performed by a known batch culture, continuous culture, fed-batch culture, etc. In this regard, the culture conditions may not be particularly limited, but an appropriate pH (e.g., pH 5 to 9, specifically pH 6 to 8, most specifically pH 6.8) may be adjusted using a basic compound (e.g., sodium hydroxide, potassium hydroxide, or ammonia) or an acidic compound (e.g., phosphoric acid or sulfuric acid), and an aerobic condition may be maintained by adding oxygen or an oxygen-comprising gas mixture to the culture. The culture temperature may be maintained at 20°C to 45°C, and specifically at 25°C to 40°C, and the cultivation may be performed for about 10 hours to about 160 hours, but is not limited thereto. The target product produced by the culture may be released into the medium or may remain within the cells.

Furthermore, in the culture medium to be used, as a carbon source, saccharides and carbohydrates (e.g., glucose, sucrose, lactose, fructose, maltose, molasses, starch, and cellulose), oils and fats (e.g., soybean oil, sunflower seed oil, peanut oil, and coconut oil), fatty acids (e.g., palmitic acid, stearic acid, and linoleic acid), alcohols (e.g., glycerol and ethanol), organic acids (e.g., acetic acid), etc. may be used alone or in a mixture, but the carbon source is not limited thereto. As a nitrogen source, a nitrogen-comprising organic compound (e.g., peptone, yeast extract, meat extract, malt extract, corn steep liquor, soybean flour, and urea) or an inorganic compound (e.g., ammonium sulfate, ammonium chloride, ammonium phosphate, ammonium carbonate, and ammonium nitrate), etc. may be used alone or in a mixture, but the nitrogen source is not limited thereto. As a phosphorus source, potassium dihydrogen phosphate, dipotassium hydrogen phosphate, a sodium-comprising salt corresponding thereto, etc. may be used alone or in a mixture, but the phosphorus source is not limited thereto. Further, the medium may also comprise essential growth-promoting materials such as other metal salts (e.g., magnesium sulfate or iron sulfate), amino acids, and vitamins.

A method of recovering the target product produced in the culture step of the present disclosure is to collect the target product from the culture using an appropriate method known in the art according to the culture method. For example, centrifugation, filtration, anion exchange chromatography, crystallization, HPLC, etc. may be used, and the target product may be recovered from the medium or microorganism using an appropriate method known in the art.

Further, the recovering step may comprise a purification process, and may be performed using an appropriate method known in the art. Therefore, the recovered target product may be in a purified form or a microbial fermentation liquid comprising the target product (Introduction to Biotechnology and Genetic Engineering, A. J. Nair., 2008).

Further, with respect to the objects of the present disclosure, the microorganism comprising the polynucleotide having the promoter activity of the present disclosure is characterized by increasing the production amount of the target product. The microorganism of the present disclosure may increase the production amount of the target product through the polynucleotide having the promoter activity of the present disclosure whereas a wild-type microorganism produces the target product in only a trace amount or does not produce the target product.

Still another aspect of the present disclosure provides use of the polynucleotide comprising any one polynucleotide sequence selected from the group consisting of SEQ ID NOS: 1 to 3 as a promoter.

### [Mode for Carrying Out the Invention]

Hereinafter, the present disclosure will be described in more detail with reference to exemplary embodiments. However, these exemplary embodiments are only for illustrating the present disclosure, and the scope of the present disclosure is not limited by these exemplary embodiments.

### Example 1: Preparation of mutant library of recombinant vectors comprising cj7 promoter sequence

To discover promoters capable of strongly inducing gene expression in microbial strains, a mutant library was prepared, based on the existing cj7 promoter (SEQ ID NO: 4: US 7662943 B2), which is known to exhibit strong activity.

First, to amplify the cj7 promoter, an expression vector pCES208-Pcj7-GFP was constructed, in which a nucleic acid molecule where the cj7 promoter (SEQ ID NO: 4) and a green fluorescent protein (GFP) were operably linked was inserted into pCES208, which is an E. *coli-Corynebacterium* shuttle vector (J.Microbiol. Biotechnol. 18:639-647, 2008). In detail, PCR was performed using p117-cj7-gfp (US 7662943 B2) as a template and primers of SEQ ID NOS: 5 and 6. The cj7-GFP fragment obtained by PCR was fusion-cloned into the pCES208 vector using an In-Fusion^{®} HD cloning kit (Clontech). The resulting plasmid was named pCES208-Pcj7-GFP. A mutant library was prepared using pCES208-Pcj7-GFP as a template.

The library was prepared using an error-prone PCR kit (clontech Diversify^{®} PCR Random Mutagenesis Kit). PCR was performed using primers of SEQ ID NOS: 7 and 8 under conditions where mutations may occur. The conditions where 1 mutation occurs per 1000 bp were as follows: pre-heating was performed at 94°C for 30 seconds by adding 40 µM (final rxn) of dGTP without MnSO₄ (8 mM), followed by 25 cycles of 94°C for 30 seconds and 68°C for 1 minute. The PCR product thus obtained was subjected to 25 cycles of denaturation at 95°C for 50 seconds, annealing at 60°C for 50 seconds, and extension at 68°C for 12 minutes using a megaprimer (500 ng to 125 ng), followed by Dpnl treatment, and transformed into *Corynebacterium glutamicum* ATCC13032 strain to prepare a recombinant vector mutant library (pCES208_Pcj7(library)-gfp).

### Example 2: Evaluation of activity of recombinant vector to induce green fluorescent protein (GFP) expression in Corynebacterium glutamicum

In order to evaluate the activity of the recombinant vector mutant library prepared in Example 1, GFP expression in the recombinant strain into which the vector was introduced was compared.

The strain transformed in Example 1 was exposed to ultraviolet light, and the fluorescent strain was first selected. Next, the promoter activity was quantitatively measured for second selection. In detail, *Corynebacterium glutamicum* ATCC13032, into which the recombinant vector pCES208_Pcj7(library)-gfp was introduced, was cultured, and the cells were obtained by centrifugation. Next, the recovered cells were suspended in a protein extraction buffer (1 mM EDTA, 3% glycerol, 1% triton-X-100 solution in PBS, pH-7.5) and the cells were disrupted by sonication. The cell lysate was centrifuged, and the supernatant containing the cell extract was collected. The protein quantity in the obtained cell extract was determined by the Bradford assay. Next, the same amount of the cell extract was irradiated with light at an excitation wavelength of 488 nm using the method of Laure Gory or the like, and fluorescence intensity was measured with light at an emission wavelength of 511 nm using the LS-50B spectrophotometer (PerkinElmer), thereby evaluating the expression degree of the GFP gene.

10 types of mutants with GFP expression levels of about 5 times to about 9 times or about 7 times to about 9 times higher than that of the cj7 promoter were selected, and the expression levels of the GFP gene in these mutants were measured by fluorescence intensity, and the results are shown in FIG. 1. Among them, the top 3 mutants with high GFP expression, M2, M7, and M9, were selected, and their green fluorescence intensities are shown in Table 1. These mutant promoters were named cj7.7, cj7.8, and cj7.9, respectively, and their sequences were analyzed and shown in Table 2.

**[Table 1]**

| Name of strain | Promoter | Fluorescence intensity | Relative fluorescence intensity |
|---|---|---|---|
| ATCC13032/pCES208 | Endogenous promoter | 0 | - |
| ATCC13032/pCES208_Pcj7_gfp | cj7 | 4269 | 100% |
| ATCC13032/pCES208_Pcj7.7_gfp | cj7.7 | 31504 | 738% |
| ATCC13032/pCES208_Pcj7.8_gfp | cj7.8 | 34839 | 816% |
| ATCC13032/pCES208_Pcj7.9_gfp | cj7.9 | 39868 | 934% |

**[Table 2]**

| Promoter | Nucleotide sequence (5'→3') | SEQ ID NO. |
|---|---|---|
| cj7.7 | AGAAACATCCCAGCGCTACTAATAGGGAGCGTTGACCTTCCTTCCA CGGACCGGTAATCGGAGTGCCTAAAACCGCATGCGGCTTAGGCTC CAAGATAGGTTCTGCGCGGCCGGGTAATGCATCTTCTTTAGCAACA AGTTGAGGGGTAGGTGCAAATAAGAACGACATAGAAATCGTCTCCT TTCTGTTTTTAATCAACATACACCACCACCTAAAAATTCCCCGACCA GCAAGTTCACAGTATTCGGGCACAATATCGTTGACAAAATATTGTTT CGGAATATAATGGGATACGTACCCAACGAAAGGAAACACTC | 1 |
| cj7.8 | AGAAACATCCCAGCGCTACTAATAGGGAGCGTTGACCTTCCTTCCA CGGACCGGTAATCGGAGTGCCTAAAACCGCATGCGGCTTAGGCTC CAAGATAGGTTCTGCGCGGCCGGGTAATGCATCTTCTTTAGCAACA AGTTGAGGGGTAGGTGCAAATAAGAACGACATAGAAATCGTCTCCT TTCTGTTTTTAATCAACATACACCACCACCTAAAAATTCCCCGACCA GCAAGTTCACAGTATTCGGGCACAATATCGTTGACAAAATATTGTTT TGTGGTATAATGGGATACGTACCCAACGAAAGGAAACACTC | 2 |
| cj7.9 | AGAAACATCCCAGCGCTACTAATAGGGAGCGTTGACCTTCCTTCCA CGGACCGGTAATCGGAGTGCCTAAAACCGCATGCGGCTTAGGCTC CAAGATAGGTTCTGCGCGGCCGGGTAATGCATCTTCTTTAGCAACA AGTTGAGGGGTAGGTGCAAATAAGAACGACATAGAAATCGTCTCCT TTCTGTTTTTAATCAACATACACCACCACCTAAAAATTCCCCGACCA GCAAGTTCACAGTATTCGGGCACAATATCGTTGCCAAAATATTGTTT TGTGGTATCATGGGATACGTACCCAACGAAAGGAAACACTC | 3 |

### Example 3: Evaluation of activity of recombinant vector to induce green fluorescent protein (GFP) expression in E.coli

### 3-1. Preparation of transformed strain

The recombinant vectors pCES208_Pcj7.7_gfp, pCES208_Pcj7.8_gfp, pCES208_Pcj7.9_gfp and pCES208_Pcj7_gfp selected in Example 2 were each transformed into E. *coli* DH5α by the heat shock method, and the transformed strains were obtained on Luria-Bertani (LB) agar medium comprising 25 mg/L of kanamycin, and named 'DH5α/pCES208_Pcj7.7_gfp', 'DH5α/pCES208_Pcj7.8_gfp', 'DH5α/pCES208_Pcj7.9_gfp' and 'DH5α/pCES208_Pcj7_gfp', respectively.

### 3-2. Examination of expression-inducing activity of transformed strain

To examine the activities of cj7.7, cj7.8, and cj7.9 promoters in E. *coli,* the transformed strains DH5α/pCES208_Pcj7.7_gfp, DH5α/pCES208_Pcj7.8_gfp, DH5α/pCES208_Pcj7.9_gfp, and DH5α/pCES208_Pcj7_gfp obtained in Example 3-1 were cultured by the following method, and GFP activities were measured.

In detail, the transformed E. *coli* strains were each inoculated into a 250 ml corner-baffled flask containing 25 ml of LB medium containing kanamycin at a volume ratio of 1:20, and cultured with shaking (200 rpm) at 37°C to the mid-growth phase (OD600=3.0). The cells were collected from the culture solution by centrifugation (5,000 rpm, 15 minutes), washed twice with a 0.1% (w/v) Tris.HCl (pH 8.0) buffer solution, suspended in the same buffer solution, and disrupted by sonication, and the supernatant was collected by centrifugation (15,000 rpm, 20 minutes), and the protein concentration was quantified by the Bradford method. The same amount of cell extract was irradiated with light at an excitation wavelength of 488 nm using the method of Laure Gory or the like, and measured with light at an emission wavelength of 511 nm using the LS-50B spectrophotometer (PerkinElmer). The expression degree of the GFP gene was measured, and shown in Table 3 below.

**[Table 3]**

| Strain | Promoter | Fluorescence intensity | Relative fluorescence intensity |
|---|---|---|---|
| DH5α/pCES208_Pcj7_gfp | cj7 | 473 | 100% |
| DH5α/pCES208_Pcj7.7_gf p | cj7.7 | 1062 | 225% |
| DH5α/pCES208_Pcj7.8_gf p | cj7.8 | 1358 | 287% |
| DH5α/pCES208_Pcj7.9_gf p | cj7.9 | 2787 | 489% |

As shown in Table 3 below, it was confirmed that in *E*. *coli,* the cj7.7, cj7.8, and cj7.9 promoters all exhibited promoter activity, and had promoter activity at least twice higher than that of the cj7 promoter, which was previously known as a strong promoter.

### Example 4: Evaluation of activity of recombinant vector to induce green fluorescent protein (GFP) expression in Bacillus subtilis

### 4-1. Construction of recombinant vector comprising cj7.7, cj7.8, or cj7.9 promoter

To construct recombinant vectors each comprising Pcj7.7, Pcj7.8, or Pcj7.9, PCR was performed using primers of SEQ ID NOS: 9 and 10 and promoter cj7.7, promoter cj7.8, and promoter cj7.9 as templates, respectively. PCR was performed by denaturation at 94°C for 5 minutes, followed by 30 cycles of denaturation at 94°C for 30 seconds, annealing at 55°C for 30 seconds, and polymerization at 72°C for 1 minute, and then polymerization at 72°C for 5 minutes, thereby obtaining Pcj7.7, Pcj7.8, and Pcj7.9, respectively.

Further, the open reading frame (ORF) of the GFP gene was obtained by PCR using pGFPuv vector (clontech, USA) as a template and primers of SEQ ID NOS: 11 and 12. PCR was performed by denaturation at 94°C for 5 minutes, followed by 30 cycles of denaturation at 94°C for 30 seconds, annealing at 55°C for 30 seconds, and polymerization at 72°C for 1 minute, and then polymerization at 72°C for 7 minutes, thereby obtaining each gene fragment comprising the ORF of GFP.

Further, in order to introduce each of Pcj7.7, Pcj7.8, and Pcj7.9, and GFP into *a Bacillus* expression vector pHT43 (Mobitec GmbH), the vector was prepared by performing PCR using the pHT43 vector as a template and primers of SEQ ID NOS: 13 and 14. PCR was performed by denaturation at 94°C for 5 minutes, followed by 30 cycles of denaturation at 94°C for 30 seconds, annealing at 55°C for 30 seconds, and polymerization at 72°C for 5 minutes, and then polymerization at 72°C for 5 minutes.

Thereafter, Pcj7.7, Pcj7.8 or Pcj7.9, and ORF of GFP gene were operably linked using an infusion enzyme to the pHT43 vector obtained by the above method, thereby preparing a recombinant vector in which Pcj7.7, Pcj7.8, or Pcj7.9 was linked to GFP, and each named 'pHT_Pcj7.7_gfp', 'pHT_Pcj7.8_gfp', and 'pHT_Pcj7.9_gfp'.

As a control group for examining the activity of the novel promoter, the previously known strong promoter P43 (Wang and Doi, J Biol Chem. 1984 Jul 10;259(13):8619-25) as a template and primers of SEQ ID NOS: 15 and 16 were used to perform PCR, thereby obtaining P43. Further, a gene fragment comprising the open reading frame (ORF) of the GFP gene was obtained by PCR using the pGFPuv vector (clontech, USA) as a template and primers of SEQ ID NOS: 17 and 12. Thereafter, in the same manner as the above, P43 and ORF of the GFP gene were operably linked to the pHT43 vector using a BD In-Fusion kit to construct a recombinant vector, in which P43 and GFP were linked, which was named 'pHT_P43_gfp'.

### 4-2. Preparation of transformed strain

The recombinant vectors constructed in Example 4-1, pHT_Pcj7.7_gfp, pHT_Pcj7.8_gfp, pHT_Pcj7.9_gfp and pHT_P43_gfp comprising the existing known strong promoter P43 were each transformed into *Bacillus subtilis* ATCC23857 by an electric pulse method (Eppendorf Protocol No. 4308 915 504), and the transformed strains were each obtained from a selection medium comprising 50 mg/L of kanamycin, and named 'ATCC23857/pHT_Pcj7.7_gfp', 'ATCC23857/pHT_Pcj7.8_gfp', 'ATCC23857/pHT_Pcj7.9_gfp' and 'ATCC23857/pHT_P43_gfp', respectively.

### 4-3. Examination of expression-inducing activity of Pcj7.7, Pcj7.8, and Pcj7.9

To examine the activities of cj7.7, cj7.8, and cj7.9 promoters in *Bacillus subtilis,* the transformed strains *Bacillus subtilis* ATCC23857/pHT_Pcj7.7_gfp, ATCC23857/pHT_Pcj7.8_gfp, ATCC23857/pHT_Pcj7.9_gfp and ATCC23857/pHT_P43_gfp obtained in Example 4-2 were cultured using the following method, and the GFP activities were measured.

In detail, the transformed *Bacillus subtilis* strains were each inoculated into a 250 ml corner-baffled flask containing 25 ml of a seed medium at a volume ratio of 1:20, and cultured with shaking (200 rpm) at 30°C to the mid-growth phase (OD600=10.0). The components of the seed medium are listed in Table 4 below. The cells were collected from the culture solution by centrifugation (5,000 rpm, 15 minutes), washed twice with a 0.1% Tris.HCl (pH8.0) buffer solution, and then suspended in the same buffer solution until the turbidity at 610 nm was approximately 160. After adding 1.25 g of glass beads per 1.5 ml of the suspension, the cells were disrupted for 6 minutes using a bead beater, and the supernatant was collected through centrifugation (15,000 rpm, 20 minutes), and the protein concentration was quantified by the Bradford method (Bradford, M.M 1976. Anal. Biochem. 72:248-254). The same amount of cell extract was irradiated with light at an excitation wavelength of 488 nm using the method of Laure Gory or the like, and the fluorescence intensity was measured with light at an emission wavelength of 511 nm using the LS-50B spectrophotometer (PerkinElmer). The expression degree of the GFP gene was assessed, and shown in Table 5 below.

**[Table 4]**

| Type of medium | Components |
|---|---|
| Seed medium | 10 g of glucose, 2.68 g of ammonium sulfate, 20 g of yeast extract, 30 g/L of corn steep liquor,4.5 g of NaH₂PO₄2H₂O, 14.6 g of K₂HPO₄, 2 g of MgSO₄7H₂O, 2 g/L of Na₂SO₄ (based on 1 liter of distilled water) |

**[Table 5]**

| Strain | Promoter | Fluorescence intensity | Relative fluorescence intensity |
|---|---|---|---|
| ATCC23857/pHT_P43_gfp | P43 | 307 | 100% |
| ATCC23857/pHT_Pci7.7_gfp | cj7.7 | 355 | 116% |
| ATCC23857/pHT_Pcj7.8_gfp | ci7.8 | 389 | 127% |
| ATCC23857/pHT_Pcj7.9_gfp | cj7.9 | 448 | 146% |

As shown in Table 5, it was confirmed that in *Bacillus subtilis,* the cj7.7, cj7.8, and cj7.9 promoters all exhibited promoter activity, and had the higher promoter activity than P43, which was previously known as a strong promoter.

The above results suggest that the mutant promoters of the present disclosure, cj7.7, cj7.8, and cj7.9 promoters, may function as general-use promoters in various host cells, not only in the genus *Corynebacterium* but also in the genus *Escherichia* and the genus *Bacillus.*

### Example 5: Evaluation of target product-producing ability

### 5-1. Evaluation of acetyl homoserine-producing ability

### 5-1-1. Construction of gapN expression vectors each comprising cj7, cj7.7, cj7.8, or cj7.9 promoter sequence

To determine the effect of the mutant promoter of cj7 on acetyl homoserine production, a vector was constructed, in which expression of the gapN gene (NADP-dependent glyceraldehyde-3-phosphate dehydrogenase derived from *Lactobacillus delbrueckii subsp. Bulgaricus*) is regulated by the mutant promoter of cj7. An amino acid sequence (SEQ ID NO: 18) and a nucleotide sequence (SEQ ID NO: 19) of the Ldb1179 gene encoding gapN derived from *Lactobacillus delbrueckii subsp. Bulgaricus* ATCC 11842 were obtained from the NIH GenBank.

In detail, PCR was performed using DNAs of the pCES208_Pcj7.7_gfp, pCES208_Pcj7.8_gfp, pCES208_Pcj7.9_gfp or pCES208_Pcj7_gfp vectors constructed in Examples 1 and 2 as templates and primers of SEQ ID NOS: 20 and 21, respectively. PCR was performed by denaturation at 94°C for 5 minutes, followed by 30 cycles of denaturation at 94°C for 30 seconds, annealing at 55°C for 30 seconds, and polymerization at 72°C for 1 minute, and then polymerization at 72°C for 5 minutes, thereby obtaining DNA fragments each comprising the Pcj7.7, Pcj7.8, Pcj7.9, or Pcj7 promoter sequence.

Further, approximately 1.43 kb of a gene fragment in which the initiation codon TTG was modified to ATG was prepared by amplification using the chromosome of *Lactobacillus delbrueckii subsp. Bulgaricus* ATCC 11842 strain as a template and primers of SEQ ID NOS: 22 and 23. At this time, PCR reaction was performed by 30 cycles of denaturation at 95°C for 30 seconds, annealing at 55°C for 30 seconds, and elongation at 72°C for 1 minute and 30 seconds. The PCR product was electrophoresed on a 0.8% agarose gel, and approximately 1.4 kb of a band was eluted and purified.

The above amplified products, cj7 promoter mutant PCR products (Pcj7.7, Pcj7.8, Pcj7.9) or Pcj7 gene fragment and ORF of *gapN* gene were mixed with pCES208 (J.Microbiol. Biotechnol. 18:639-647, 2008) digested with BamHI/Sall restriction enzymes, which is an *E*. *coli-Corynebacterium* shuttle vector, thereby constructing recombinant vectors, in which Pcj7.7, Pcj7.8, Pcj7.9, or Pcj7 were linked to *gapN,* using the In-Fusion^{®} HD cloning kit (clontech), and named 'pCES_Pcj7.7_gapN', 'pCES_Pcj7.8_gapN', 'pCES_Pcj7.9_gapN' and 'pCES_Pcj7_gapN', respectively.

### Example 5-1-2. Preparation of transformed strains

The recombinant vectors 'pCES_Pcj7.7_gapN', 'pCES_Pcj7.8_gapN', 'pCES_Pcj7.9_gapN', and 'pCES_Pcj7_gapN' prepared in Example 5-1-1 were each transformed into *Corynebacterium glutamicum* KCCM12634P (US 2023-0340549 A1), which is an acetyl homoserine-producing strain, by an electric pulse method, and the transformed strains were obtained from a selection medium comprising 25 mg/L of kanamycin, and named 'KCCM12634P::Pcj7.7_gapN', 'KCCM12634P::Pcj7.8_gapN', 'KCCM12634P::Pcj7.9_gapN', and 'KCCM12634P::Pcj7_gapN', respectively.

### Example 5-1-3. Evaluation of O-acetyl homoserine-producing ability of transformed strains

The transformed strains prepared in Example 5-1-2 were cultured by the following method, respectively and then the O-acetyl homoserine-producing ability was measured. The 'KCCM12634P::Pcj7_gapN' strain was used as a control group.

In detail, one inoculation loop of the strain was inoculated into a 250 ml corner-baffled flask containing 25 ml of a production medium, and cultured at 37°C for 20 hours with shaking at 200 rpm. The composition of the production medium is as follows.

### <Production medium (pH 7.2)>

30 g of glucose, 2 g of KH₂PO₄, 3 g of urea, 40 g of (NH₄)₂SO₄, 2.5 g of peptone, 5 g (10 ml) of CSL (Sigma), 0.5 g of MgSO₄·7H₂O, 400 mg of methionine, 400 mg of leucine, 20 g of CaCO₃ (based on 1 liter of distilled water)

After completing the culture, the O-acetyl homoserine-producing ability was measured by HPLC. Based on the strain comprising the cj7 promoter, the increase rate of O-acetyl homoserine concentration in the culture medium of each tested strain is as shown in Table 6 below.

**[Table 6]**

| Name of strain | Increase rate of O-acetyl homoserine concentration (%) |
|---|---|
| KCCM12634P::pCES208_Pcj7 _gapN | 100% |
| KCCM12634P::pCES208_Pcj7.7_gapN | 109.67% |
| KCCM12634P::pCES208_Pcj7.8_gapN | 116.12% |
| KCCM12634P::pCES208_Pcj7.9_gapN | 119.35% |

As shown in the above table, it was confirmed that when the mutant promoter of cj7 was introduced, the activity of gapN increased and the production amount of acetyl homoserine increased, suggesting that the mutant promoter of cj7 of the present disclosure was effective in increasing the production amount of O-acetyl homoserine.

### 5-2. Evaluation of isoleucine-producing ability

### 5-2-1. Preparation of Corynebacterium glutamicum transformed strains having isoleucine-producing ability

In order to examine the effect of the mutant promoter of cj7 on isoleucine-producing ability, among the four vectors constructed in Example 5-1-1, pCES_Pcj7.9_gapN which showed excellent performance in the evaluation of GFP expression intensity and acetyl homoserine-producing ability and pCES_Pcj7_gapN were transformed into *Corynebacterium glutamicum* KCCM12739P (CA10-3101, US 2023-0098971 A1), which is an isoleucine-producing strain, by electroporation to construct isoleucine-producing strains into which the cj7 promoter or the mutant promoter of cj7 was introduced. The strains thus constructed were named KCCM12739P::pCES208_Pcj7_gapN, and KCCM12739P::pCES208_Pcj7.9_gapN, respectively.

### 5-2-2. Evaluation of isoleucine-producing ability of transformed strains

The prepared strains were cultured by the following method, and then the isoleucine-producing ability was compared.

Each strain was inoculated into a 250 ml corner-baffled flask comprising 25 ml of a production medium, and cultured at 32°C for 60 hours with shaking at 200 rpm. The composition of the production medium is as follows.

### <Production medium (pH 7.2)>

10% glucose, 0.2% yeast extract, 1.6% ammonium sulfate, 0.1% potassium phosphate monobasic, 0.1% magnesium sulfate heptahydrate, 10 mg/L of iron sulfate heptahydrate, 10 mg/L of manganese sulfate monohydrate, 200 µg/L of biotin (based on 1 liter of distilled water)

After completing the culture, the isoleucine-producing ability was measured by HPLC. Based on the strain comprising the cj7 promoter, the increase rate of L-isoleucine concentration in the culture medium of each tested strain is as shown in Table 7 below.

**[Table 7]**

| Name of strain | Increase rate of L-isoleucine concentration (%) |
|---|---|
| KCCM12739P::pCES208_Pcj7 _gapN | 100% |
| KCCM12739P::pCES208_Pcj7.9_gapN | 111% |

As shown in the above table, it was confirmed that when the mutant promoter of cj7 was introduced, the activity of gapN increased and the production amount of isoleucine increased, suggesting that the mutant promoter of cj7 of the present disclosure was effective in increasing the production amount of L-isoleucine.

### 5-3. Evaluation of valine-producing ability

### 5-3-1. Construction of ilvE expression vectors each comprising cj7 or cj7.9 promoter sequence

To determine the effect of the mutant promoter of cj7 on valine production, a vector was constructed, in which ilvE (Ncgl2123, SEQ ID NO: 24) encoding branched-chain amino acid aminotransferase, which is a key gene for valine biosynthesis, is expressed by Pcj7.9 and Pcj7.

In detail, PCR was performed using DNA of the pCES208_Pcj7.9_gfp or pCES208_Pcj7_gfp vector constructed in Examples 1 and 2 as a template and primers of SEQ ID NOS: 25 and 26, respectively. PCR was performed by denaturation at 94°C for 5 minutes, followed by 30 cycles of denaturation at 94°C for 30 seconds, annealing at 55°C for 30 seconds, and polymerization at 72°C for 1 minute, and then polymerization at 72°C for 5 minutes, thereby obtaining DNA fragments each comprising the Pcj7.9 or Pcj7 promoter sequence.

Further, approximately 1.1 kb of ilvE gene fragment was prepared by PCR amplification using the chromosome of *Corynebacterium glutamicum* ATCC14067 as a template and primers of SEQ ID NOS: 27 and 28. PCR reaction was performed by 30 cycles of denaturation at 95°C for 30 seconds, annealing at 55°C for 30 seconds, and elongation at 72°C for 1 minute and 30 seconds. The PCR product was electrophoresed on a 0.8% agarose gel, and approximately 1.1 kb of a band was eluted and purified.

The above amplified product, Pcj7.9 or Pcj7 gene fragment and ORF of *ilvE* gene were mixed with the prepared *E*. *coli-Corynebacterium* shuttle vector pCES208 digested with EcoRV/Sall restriction enzymes (J.Microbiol. Biotechnol. 18:639-647, 2008), thereby constructing recombinant vectors, in which Pcj7.9 or Pcj7 was linked to *ilvE,* using the In-Fusion^{®} HD cloning kit (clontech), and named 'pCES_Pcj7.9_ilvE' and 'pCES_Pcj7_ilvE', respectively.

### 5-3-2. Preparation of transformed Corynebacterium glutamicum strains having valine-producing ability

The two recombinant vectors 'pCES_Pcj7.9_ilvE' and 'pCES208_Pcj7_ilvE' constructed in Example 5-3-1 were each transformed into *Corynebacterium glutamicum* KCCM11201P (US 8465962 B2), which is a valine-producing strain, by electroporation, and the transformed strains were obtained from a selection medium comprising 25 mg/L of kanamycin, and named KCCM11201P::pCES208_Pcj7.9_ilvE and KCCM11201P::pCES208_Pcj7_ilvE, respectively.

### 5-3-3. Evaluation of valine-producing ability of transformed strains

The prepared strains were cultured by the following method, and then the valine-producing ability was compared.

Each strain was inoculated into a 250 ml corner-baffled flask containing 25 ml of a production medium, and cultured at 30°C for 72 hours with shaking at 200 rpm. The composition of the production medium is as follows.

### <Production medium (pH 7.2)>

100 g of glucose, 40 g of ammonium sulfate, 2.5 g of soy protein, 5 g of corn steep solids, 3 g of urea, 1 g of potassium phosphate dibasic, 0.5 g of magnesium sulfate heptahydrate, 100 µg of biotin, 1 mg of thiamine-HCl, 2 mg of calcium pantothenate, 3 mg of nicotinamide, 30 g of calcium carbonate (based on 1 liter of distilled water)

After completing the culture, the valine-producing ability was measured by HPLC. Based on the strain comprising the cj7 promoter, the increase rate of L-valine concentration in the culture medium of each tested strain is as shown in Table 8 below.

**[Table 8]**

| Name of strain | Increase rate of L-valine concentration (%) |
|---|---|
| KCCM11201P:: pCES208_Pcj7_ilvE | 100% |
| KCCM11201 P:: pCES208_Pcj7.9_ilvE | 115% |

As shown in the above table, it was confirmed that when the mutant promoter of cj7 was introduced, the activity of ilvE increased and the production amount of valine increased, suggesting that the mutant promoter of cj7 of the present disclosure was effective in increasing the production amount of L-valine.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A polynucleotide having promoter activity, the polynucleotide comprising any one polynucleotide sequence selected from the group consisting of SEQ ID NOS: 1 to 3.

2. An expression cassette comprising the polynucleotide of claim 1 and a target gene.

3. A microorganism comprising the polynucleotide of claim 1 or the expression cassette of claim 2.

4. The microorganism of claim 3, wherein the microorganism is a microorganism of the genus *Corynebacterium,* the genus *Escherichia,* or the genus *Bacillus.*

5. A method of producing a target product, the method comprising the step of culturing the microorganism of claim 3 in a medium.

6. The method of claim 5, further comprising the step of recovering the target product from the microorganism or the medium in which the microorganism is cultured.

7. The method of claim 5, wherein the target product is one or more selected from the group consisting of amino acids, nucleic acids, vitamins, proteins, fatty acids, and organic acids.

8. The method of claim 5, wherein the microorganism is a microorganism of the genus *Corynebacterium,* the genus *Escherichia,* or the genus *Bacillus.*

9. Use of a polynucleotide comprising any one polynucleotide sequence selected from the group consisting of SEQ ID NOS: 1 to 3 as a promoter.
